# EUROPEAN PATENT APPLICATION

(11) **EP 1 854 811 A1**
(43) Date of publication of application: **14.11.2007**
(21) Application number: 06714819.7
(22) Date of filing: 28.02.2006
(51) Int. Cl.: C07K 16/40, G01N 33/537

(54) **ASSAY METHOD FOR HUMAN OROTATE PHOSPHORIBOSYLTRANSFERASE PROTEIN**

(30) Priority: 03.03.2005 JP 2005059221
(71) Applicant: TAIHO PHARMACEUTICAL COMPANY LIMITED, Chiyoda-ku, Tokyo 101-8444 (JP)
(72) Inventor: SAKAMOTO, Kazuki, Taiho Pharmaceutical Co., Ltd, Tokushima-shi, Tokushima 7310132 (JP); SUGIMOTO, Yoshikazu, Taiho Pharmaceutical Co., Ltd, Tokushima-shi, Tokushima 7310132 (JP)
(74) Representative: Blodig, Wolfgang
(86) International application number: PCT/JP2006/303682
(87) International publication number: WO 2006/093115

(57) **Abstract**

The method for assaying human orotate phosphoribosyltransferase protein includes employing, in combination, an anti-human orotate phosphoribosyltransferase antibody which recognizes an epitope present in a region of 86th to 108th amino acid residues from the N-terminus of human orotate phosphoribosyltransferase, and an anti-human orotate phosphoribosyltransferase antibody which recognizes an epitope present in a region of 454th to 474th amino acid residues from the N-terminus of human orotate phosphoribosyltransferase.

## Description

### Technical Field

The present invention relates to a novel antibody to human orotate phosphoribosyltransferase, and to a method for assaying human orotate phosphoribosyltransferase protein by use of the antibody.

### Background Art

Orotate phosphoribosyltransferase (EC 2.4.2.10, hereinafter may be referred to as "OPRT") is an enzyme which catalyzes reaction for forming uridyl monophosphate (UMP) from orotic acid. OPRT plays a role in supplying pyrimidine nucleotide, which is essential for the synthesis of nucleic acid, and is an important rate-determining enzyme for a nucleic acid precursor supply pathway. Therefore, OPRT is known to exhibit high activity in tumor tissue or the gastrointestinal epithelium, where cells are actively proliferating.

Meanwhile, 5-fluorouracil anticancer agent is activated by OPRT as a rate-determining enzyme, thereby exhibiting antitumor effect. Thus, as has been known, 5-fluorouracil anticancer agent exhibits considerable antitumor effect and remarkable life-prolonging effect in patients exhibiting high tumor cell OPRT level, but exhibits insignificant antitumor effect in patients exhibiting a low OPRT level (see Non-Patent Document 1). Therefore, for the treatment of tumor patients, determination of the OPRT expression level of the extirpated tumor sample is of great importance, since, for example, the thus-determined OPRT expression level is used as an index for selecting the treatment method, therefore or the anticancer agent to be administered to patients in need thereof.

Conventionally, the human OPRT level of tumor tissue has been quantitatively determined by assaying mRNA level or enzyme activity. However, the thus-assayed mRNA level may fail to sufficiently reflect the amounts of the protein or enzyme activity, due to, for example, post-transcriptional regulatory mechanisms. Quantitative determination of OPRT level through enzyme activity assay generally employs a radiolabeled substrate, and thus the process thereof becomes very intricate. From the viewpoint of application of a human OPRT quantitative determination method to clinical diagnosis or treatment, it is important that the method is convenient and accurate. Therefore, keen demand has arisen for development of such a quantitative determination method.
Non-Patent Document 1: Br. J. Cancer., 2003, Oct., 20; 89 (8): 1486-92.

### Disclosure of the Invention

### Problems to be Solved by the Invention

In view of the foregoing, an object of the present invention is to provide a method for conveniently and accurately assaying human OPRT.

### Means for Solving the Problems

In order to achieve the aforementioned object, the present inventors have prepared polyclonal antibodies to the entire human OPRT and antibodies to various oligopeptide fragments producible from an amino acid sequence of human OPRT, and have designed assay systems employing these antibodies in combination. As a result, the inventors have found that a human OPRT immunoassay system containing, in combination, an antibody which recognizes an epitope present in a region of 86th to 108th amino acid residues from the N-terminus of human OPRT, and an antibody which recognizes an epitope present in a region of 454th to 474th amino acid residues from the N-terminus of human OPRT can conveniently and accurately assay a human OPRT protein at remarkably high sensitivity, as compared with an assay system employing an antibody(s) other than the aforementioned antibodies. The present invention has been accomplished on the basis of this finding.

Accordingly, the present invention provides an anti-human OPRT antibody which recognizes an epitope present in a region of 86th to 108th amino acid residues from the N-terminus of human OPRT.
The present invention also provides an anti-human OPRT antibody which recognizes an epitope present in a region of 454th to 474th amino acid residues from the N-terminus of human OPRT.
The present invention also provides a human OPRT protein assay kit comprising an anti-human OPRT antibody which recognizes an epitope present in a region of 86th to 108th amino acid residues from the N-terminus of human OPRT; and an anti-human OPRT antibody which recognizes an epitope present in a region of 454th to 474th amino acid residues from the N-terminus of human OPRT.
The present invention also provides a method for assaying human OPRT protein, the method comprising employing, in combination, an anti-human OPRT antibody which recognizes an epitope present in a region of 86th to 108th amino acid residues from the N-terminus of human OPRT, and an anti-OPRT antibody which recognizes an epitope present in a region of 454th to 474th amino acid residues from the N-terminus of human OPRT.
The amino acid sequence of human OPRT protein is as described in Proceeding of the National Academy of Sciences of the United States of America, Vol. 85, No. 6, 1988, 1754-1758.

### Effects of the Invention

According to the method of the present invention, human OPRT protein level can be quantitatively determined conveniently and accurately, as compared with the case in which an antibody to the entire human OPRT serving as an antigen is used, or the case in which an anti-OPRT antibody which recognizes an epitope present in a region of 428th to 446th amino acid residues from the N-terminus of human OPRT is used. When the human OPRT protein level in a sample is quantitatively determined through the assay method of the present invention, the thus-determined OPRT level can be employed for cancer diagnosis or for prediction of a therapeutic effect. In addition, the OPRT level can be employed for selecting a treatment method or determining whether or not an anticancer agent can be administered.

### Brief Description of the Drawings

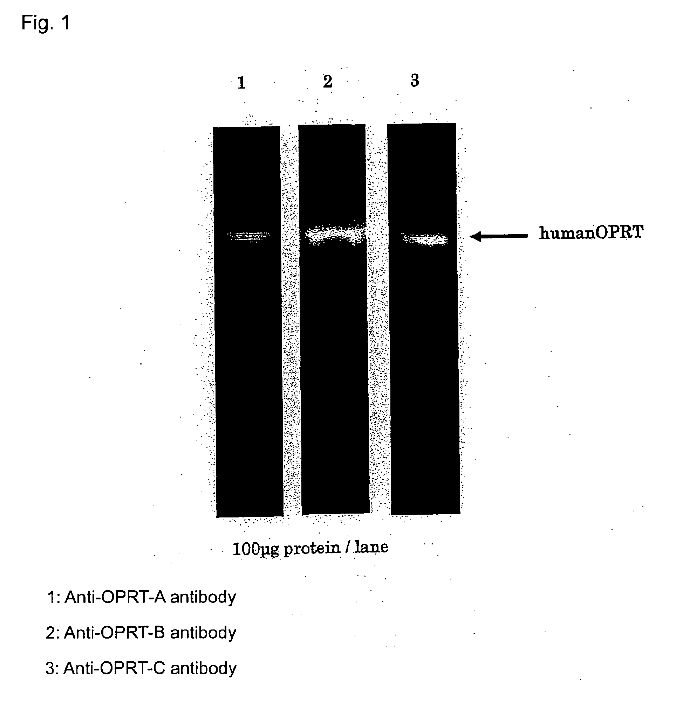

[Fig. 1] Fig. 1 shows the results of western blotting of anti-OPRT antibodies.

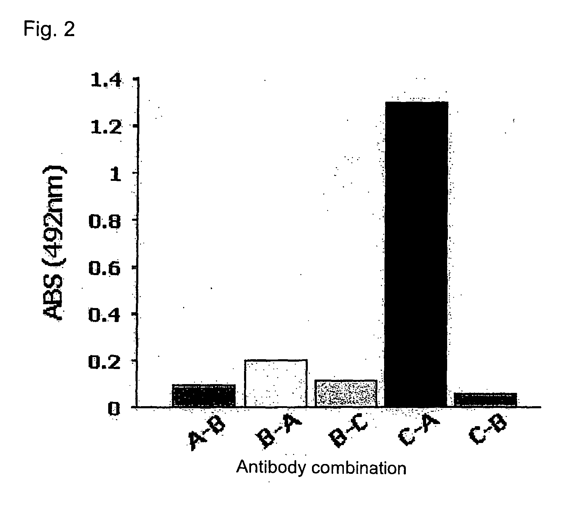

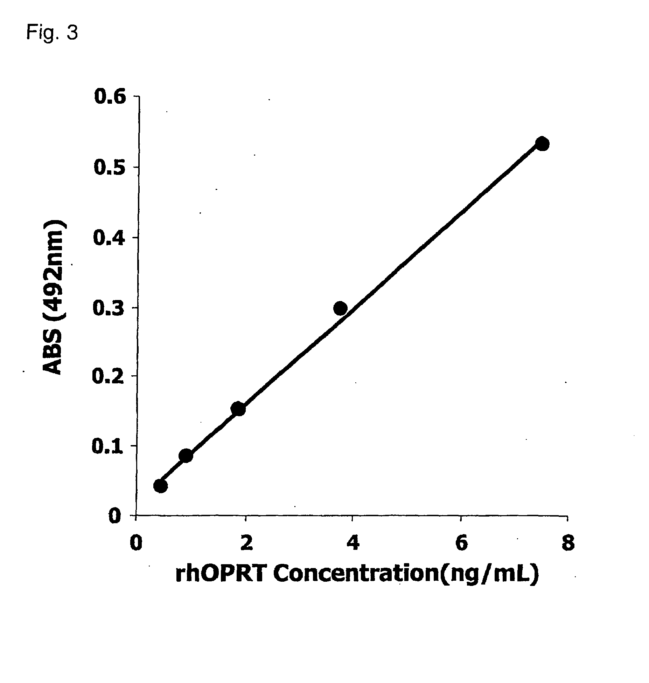

[Fig. 2] Fig. 2 shows the results of sandwich ELISA systems, each employing a combination of two species from anti-OPRT-A antibody, anti-OPRT-B antibody, and anti-OPRT-C antibody. In Fig. 2, "A-B" represents a combination of anti-OPRT-A antibody (immobilized antibody) and anti-OPRT-B antibody (detection antibody); "B-A" represents a combination of anti-OPRT-B antibody (immobilized antibody) and anti-OPRT-A antibody (detection antibody); "B-C" represents a combination of anti-OPRT-B antibody (immobilized antibody) and anti-OPRT-C antibody (detection antibody); "C-A" represents a combination of anti-OPRT-C antibody (immobilized antibody) and anti-OPRT-A antibody (detection antibody); and "C-B" represents a combination of anti-OPRT-C antibody (immobilized antibody) and anti-OPRT-B antibody (detection antibody). [Fig. 3] Fig. 3 shows a calibration curve for an ELISA system employing anti-OPRT-C antibody as an immobilized antibody and anti-OPRT-A antibody as a detection antibody. Best Modes for Carrying Out the Invention

There may be employed, as an antigen for an anti-human OPRT antibody of the present invention which recognizes an epitope present in a region of 86th to 108th amino acid residues from the N-terminus of human OPRT protein, a synthetic peptide having a sequence of 86th to 108th amino acid residues from the N-terminus of human OPRT protein, or a synthetic peptide having a stretch of amino acid residues accounting for 80% or more of the 86th to 108th amino acid residues. Hereinafter, an antibody prepared by use of such an antigen may be referred to as "anti-OPRT-A antibody."

There may be employed, as an antigen for an anti-human OPRT antibody of the present invention which recognizes an epitope present in a region of 454th to 474th amino acid residues from the N-terminus of human OPRT protein, a synthetic peptide having a sequence of 454th to 474th amino acid residues from the N-terminus of human OPRT protein, or a synthetic peptide having a stretch of amino acid residues accounting for 80% or more of the 454th to 474th amino acid residues. Hereinafter, an antibody prepared by use of such an antigen may be referred to as "anti-OPRT-C antibody."

When any of the aforementioned synthetic peptides is employed as an antigen, for the purpose of promoting immune response, the antigen may be mixed with an adjuvant such as Freund's adjuvant, or may be bound to a carrier such as bovine thyroglobulin, BSA (bovine serum albumin), or KLH (keyhole limpet hemocyanin).

No particular limitation is imposed on the anti-human OPRT antibodies of the present invention, so long as they recognize an epitope present in any of the aforementioned amino acid sequence regions. The anti-human OPRT antibodies may be monoclonal or polyclonal.
When an anti-human OPRT polyclonal antibody is produced by use of any of the aforementioned antigens, the following procedure may be employed. Specifically, an animal (e.g., rabbit, rat, mouse, or goat) is immunized as many times as required for immunization, through a customary method, with any of the aforementioned antigens, and antiserum containing an anti-human OPRT polyclonal antibody is collected from the immunized animal. Preferably, a rabbit is immunized twice every three weeks with an antigen produced by binding KLH to any of the aforementioned synthetic peptides. The thus-produced anti-OPRT antibody may be purified from the thus-collected antiserum through a customary antibody purification technique; for example, affinity chromatography, ammonium sulfate precipitation, ion-exchange column chromatography, molecular sieve column chromatography (gel filtration), or protein A column chromatography. These purification techniques may be employed in combination or in a repeated manner for the purpose of enhancing the purity of the antibody.
When affinity chromatography (antigen-immobilized column) is carried out, any of the aforementioned synthetic peptides is immobilized onto a column, and the antiserum is applied to the column so that an anti-OPRT antibody is adsorbed onto the column, followed by elution of the anti-OPRT antibody with an eluent. The antibody is collected, whereby a high-purity anti-OPRT antibody can be produced.

For preparation of an anti-human OPRT monoclonal antibody by use of any of the aforementioned antigens, a hybridoma producing the anti-human OPRT monoclonal antibody may be produced as described below. Such a hybridoma may be produced through, for example, the following procedure. Specifically, a mammal (e.g., mouse or rat) or a bird is immunized with any of the aforementioned antigens, and spleen cells collected therefrom are fused with myeloma cells of a mammal (e.g., mouse or rat) through the basic method of Koler and Milstein [see Nature, Vol. 256, page 495 (1975)], followed by culturing in a selection medium. Immunization in the production of the hybridoma is performed through, for example, the following procedure. Specifically, the thus-produced antigen is dissolved in phosphate buffer, physiological saline, or the like, and, if necessary, mixed with an adjuvant; and the mixture is administered to an animal via, for example, a subcutaneous, intrasplenic, intraperitoneal, or intravenous route several times every one to three weeks until antibody titer is sufficiently increased. Examples of myeloma cell lines employed in cell fusion include mouse P3-NS-1/1Ag4.1, P3-X63-Ag8.653, Sp2/0Ag14, and rat YB2/0. During cell fusion, a fusion promoter such as polyethylene glycol or Sendai virus may be employed, or electrical pulse may also be employed. Since myeloma cells employed for cell fusion are 8-azaguanine-resistant cells and lack hypoxanthine-guanine-phosphoribosyltransferase required for the salvage pathway of nucleotide biosynthesis, the cells fail to synthesize a nucleotide in an HAT medium (i.e., a medium containing hypoxanthine, aminopterin, and thymidine) and thus cannot survive. Therefore, through culturing in the HAT medium for one to two weeks after cell fusion, only a splenocyte-myeloma-fused hybridoma can be selected

The thus-produced anti-human OPRT antibody of the present invention is useful for immunological assay of human OPRT protein, and can be applied to, for example, sandwich ELISA, competitive radioimmunoassay, enzyme immunoassay, and immunochromatography. Of these, sandwich ELISA is particularly preferred.

The human OPRT protein assay kit of the present invention contains anti-OPRT-A antibody and anti-OPRT-C antibody. When sandwich ELISA is carried out by use of these two antibodies, one of the anti-OPRT antibodies is immobilized onto a support (immobilized antibody), and the other anti-OPRT antibody is employed as a labeled antibody (detection antibody). Preferably, anti-OPRT-C antibody is employed as an immobilized antibody, and anti-OPRT-A antibody is employed as a detection antibody.

In the case of immunoassay; for example, sandwich ELISA, one of the anti-OPRT antibodies is immobilized onto a support such as an ELISA plate; a test sample is reacted with the anti-OPRT antibody; the test sample is further reacted with the other anti-OPRT antibody (i.e., labeled anti-OPRT antibody), followed by washing; and the amount of the labeled antibody bound to the test sample through sandwich immunoreaction is assayed.

No particular limitation is imposed on the test sample employed in the present invention, so long as it is a human tissue or body fluid in which OPRT may be present. Examples of the test sample include tumor tissue, gastrointestinal tissue, blood, and lymph. Among them, tumor tissue is particularly preferred.

Examples of the support which may be employed in the present invention include insoluble polysaccharides such as agarose and cellulose; synthetic resins such as silicone resin, polystyrene resin, polyacrylamide resin, nylon resin, and polycarbonate resin; and insoluble supports such as glass. Such a support may be employed in the form of, for example, beads or plate. When a support is in the form of beads, a column or the like charged with the beads may be employed. When a support is in the form of plate, for example, a multiwell plate (e.g., a 96-well multiwell plate) or a biosensor chip may be employed. Binding of an anti-OPRT antibody to a support may be carried out through a generally employed technique such as chemical binding or physical adsorption. Such a support may be a commercially available one.

Reaction between the anti-OPRT antibody and the test sample is generally performed in the buffer. Examples of the buffer employed include phosphate buffer, Tris buffer, citrate buffer, borate buffer, and carbonate buffer. Incubation is performed under, for example, the following conditions: 4°C to room temperature and one hour to 24 hours. For washing after incubation, any substance may be employed so long as it does not inhibit binding of the anti-OPRT antibody to human OPRT protein contained in a test sample, and examples of the substance include the buffer containing surfactant (e.g., Tween 20).

In the human OPRT protein assay method of the present invention, in addition to a test sample which is assayed for detection of human OPRT protein, a control sample may be provided. Examples of the control sample include a negative control sample containing no human OPRT protein, and a positive control sample containing human OPRT protein. In this case, human OPRT protein contained in a test sample can be detected by comparing the results obtained through assay of the test sample with the results obtained through assay of a negative control sample containing no human OPRT protein, or with the results obtained through assay of positive control sample containing human OPRT protein. Meanwhile, the amount of human OPRT protein contained in a test sample can be quantitatively determined from numerical data of the test sample on the basis of the standard curve, the standard curve being prepared by use of numerical data obtained through assay of a series of concentration-graded control samples.

Labeling of the anti-OPRT antibody may be performed through a generally known method. Examples of labeling substances which may be employed include labeling substances known to those skilled in the art, such as fluorescent dyes, enzymes, coenzymes, chemiluminescent substances, and radioactive substances. Specific examples include radioisotopes (e.g., ³²P, ¹⁴C, ¹²⁵I, ³H, and ¹³¹I), fluorescein, rhodamine, dansyl chloride, umbelliferone, luciferase, peroxidase, alkaline phosphatase, β-galactosidase, β-glucosidase, horseradish peroxidase, glucoamylase, lysozyme, saccharide oxidase, microperoxidase, and biotin. When biotin is employed as a labeling substance, preferably, after addition of a biotin-labeled antibody, an avidin to which an enzyme was bound (e.g., alkaline phosphatase) is further added. Binding of a labeling substance to an anti-OPRT antibody may be carried out through a known method such as the glutaraldehyde method, the maleimide method, the pyridyl disulfide method, or the periodate method.

Specifically, a solution containing one of the aforementioned anti-OPRT antibodies is added to a support (e.g., a plate), and the anti-OPRT antibody is immobilized onto the support. The plate is washed, and then blocked with, for example, BSA, gelatin, or albumin, so as to prevent non-specific protein binding. The plate is washed again, and a test sample is added to the plate. After incubation, the plate is washed, and the other anti-OPRT antibody (i.e., labeled anti-OPRT antibody) is added thereto. After appropriate incubation, the plate is washed, and the labeled anti-OPRT antibody remaining on the plate is detected. The labeled antibody can be detected through a method known to those skilled in the art. For example, in the case of labeling with radioactive substance, the labeled antibody can be detected through liquid scintillation or RIA. In the case of labeling with an enzyme, a substrate for the enzyme is added, and enzymatic change of the substrate (e.g., color development) can be detected by means of an absorption spectrometer. Specific examples of the substrate include 2,2-azinobis(3-ethylbenzothiazoline-6-sulfonic acid) diammonium salt (ABTS), o-phenylenediamine, and 3,3',5,5'-tetramethylbenzidine (TME). In the case of labeling with a fluorescent substance, the labeled antibody can be detected by means of a fluorometer.

### Examples

The present invention will next be described in more detail by way of examples, which should not be construed as limiting the invention thereto.

Comparative Example (quantitative determination of OPRT through ELISA employing anti-rhOPRT antibody)

### (1) Production of antigen

Recombinant human OPRT (rhOPRT) was produced through culturing of *Escherichia coli* and employed as an antigen.
The rhOPRT was produced through the following procedure. The full-length cDNA of human OPRT was cloned through PCR, and a plasmid designed for expressing a fusion protein of glutathione S-transferase (GST) and rhOPRT was produced. The plasmid was introduced into *Escherichia coli* BL21, and the *Escherichia coli* was subjected to shaking culture overnight in an LB medium (product of Wako Pure Chemical Industries, Ltd.) (100 mL) in the presence of ampicillin (100 µg/mL) at 37°C. The culture (10 mL) was transferred into an Erlenmeyer flask containing an LB medium (1 L), followed by further culturing at 37°C for four hours. Cells were collected through centrifugation, and were suspended in a buffer for collecting cells (50 mM Tris, 8 M urea, 1 mM PMSF, 5 mM EDTA, 5 mM DTT, pH 7.4) (100 mL), followed by gentle stirring at 4°C for 30 minutes. The suspension was subjected to ultrasonic disruption at 4°C, followed by centrifugation at 15,000 x g and 4°C for 30 minutes. Thereafter, the urea concentration of the supernatant was gradually reduced to 1 M through dialysis, followed by refolding treatment. The resultant product was caused to pass through a column charged with glutathione (GSH)-Sepharose (product of Sigma) (2 mL), and washed with a washing liquid (20 mM Tris, 1 M urea, 5 mM EDTA, pH 7.4) (10 mL). GST-rhOPRT was eluted with an elution buffer (50 mM GSH, 50 mM Tris, pH 9.6), to thereby yield an rhOPRT solution.

### (2) Immunization

Rabbits (Japanese white) were immunized with GST-rhOPRT (i.e., an immunogen) by use of a complete Freund's adjuvant (FCA) or an incomplete Freund's adjuvant (FIA) in the following manner.
First immunization: GST-rhOPRT 0.2 mg + FCA S.C.
Second to eighth immunizations: GST-rhOPRT 0.5 mg + FIA S.C.

### (3) Purification of antiserum

Whole blood was drawn from each of the thus-immunized rabbits, and then subjected to centrifugation at 1,500 x g and 4°C for five minutes, to thereby isolate serum.
Antiserum (20 mL) was diluted two-fold with PBS(-) to 40 mL. The diluted antiserum mixture was applied to a GST-protein-immobilized column, and a GST-reactive antibody was adsorbed onto the column. Thereafter, the flow-through liquid was caused to pass through a protein-A-immobilized column, and an antibody (IgG) was allowed to bind to protein A, followed by adequate washing with PBS(-) until no absorbance was detected at 280 nm. Thereafter, 0.2 M glycine-HCl (pH 2.5) was caused to pass through the column, to thereby elute the antibody adsorbed onto the antigen. In this case, for pH neutralization, 1 M Tris had been added in advance to a test tube which is employed for receiving the eluted antibody, to thereby prevent denaturation of the thus-collected antibody. The thus-obtained antibody fraction was dialyzed against PBS(-) at 4°C overnight, and the resultant fraction was provided as a purified antibody.

### (4) Quantitative determination of OPRT contained in human tumor cells through sandwich ELISA

An anti-rhOPRT antibody was diluted with 0.1 mM carbonate buffer (pH 9.6) to 5.0 µg/mL, and the thus-diluted antibody (0.1 mL) was dispensed into a 96-well ELISA plate. The plate was sealed and placed in an incubator at 4°C overnight for coating, to thereby yield an antibody-immobilized support. The 96-well plate was washed twice with PBS(-) supplemented with 0.05% Tween 20, and subsequently 0.1%-BSA-containing PBS(-) (0.1 mL) was added to the plate for blocking of non-specific adsorption. After the plate had been washed twice with a washing liquid, a three-, nine-, or 27-fold diluted homogenate of human gastric cancer-derived TMK1 cells (0.2 mL of protein extract prepared from 5 × 10⁶ cells) (0.1 mL) was added to the plate, and reaction was allowed to proceed at room temperature for one hour. Subsequently, the plate was washed five times with a washing liquid, and then a peroxidase-labeled anti-rhOPRT antibody which had been diluted with a diluent to 0.5 µg/mL was dispensed into the plate (0.1 mL/well), to thereby allow reaction to proceed at room temperature for 30 minutes. Subsequently, the plate was washed seven times with a washing liquid, and then a color-developing liquid (i.e., a 0.1 M phosphate-citrate buffer (pH 5.1) (0.1 mL) containing o-phenylenediamine (1.3 mg/mL), 0.01% aqueous hydrogen peroxide, and 1 mM EDTA) was added to the plate, to thereby allow enzymatic reaction to proceed in the dark at room temperature for 30 minutes. Finally, reaction was stopped by adding 0.1 M sulfuric acid (0.1 mL), and absorbance at 492 nm was measured. The results are shown in Table 1.

**[Table 1]**

| Detection of OPRT through sandwich ELISA employing anti-rhOPRT antibody | |
|---|---|
| Dilution factor | Absorbance (492 nm) |
| 1/3 | 0.085 |
| 1/9 | 0.055 |
| 1/27 | 0.15 |

The measured absorbance (492 nm) values are extremely small, and no correlation was found to exhibit with respect to the dilution factor. This indicated that an anti-rhOPRT antibody produced by use of the full-length cDNA of OPRT was not suitable for sandwich ELISA.

### Example 1 (production of antibody)

Anti-OPRT polyclonal antibodies were produced through the following procedure.

### (1) Peptide synthesis

There were synthesized artificial peptides having the below-described peptide sequences: a peptide having a sequence of 86th to 108th amino acid residues from the N-terminus of human OPRT; a peptide having a sequence of 428th to 446th amino acid residues from the N-terminus of human OPRT; and a peptide having a sequence of 454th to 474th amino acid residues from the N-terminus of human OPRT.

A peptide having a sequence of 86th to 108th amino acid residues from the N-terminus of human OPRT (antigen name: OPRT-A)
Cys-Ser-Thr-Asn-Gln-Ile-Pro-Met-Leu-Ile-Arg-Arg-Lys-Glu-Thr-Lys-Asp-Tyr-Gly-Thr-Lys-Arg-Leu (SEQ ID NO: 1)

A peptide having a sequence of 428th to 446th amino acid residues from the N-terminus of human OPRT (antigen name: OPRT-B)
Cys-Leu-Gly-Gln-Gln-Tyr-Asn-Ser-Pro-Gln-Glu-Val-Ile-Gly-Lys-Arg-Gly-Ser-Asp-Ile (SEQ ID NO: 2)

A peptide having a sequence of 454th to 474th amino acid residues from the N-terminus of human OPRT (antigen name: OPRT-C)
Cys-Ile-Ser-Ala-Ala-Asp-Arg-Leu-Glu-Ala-Ala-Glu-Met-Tyr-Arg-Lys-Ala-Ala-Trp-Glu-Ala-Tyr (SEQ ID NO: 3)

### (2) Preparation of antigen conjugate

From each of the above-produced artificial peptides (4 mg) and maleimide-activated KLH (Pierce) (2 mg), a corresponding peptide-KLH conjugate is produced.

### (3) Immunization

Rabbits (Japanese white) were immunized with each artificial peptide-KLH (i.e., an immunogen) by use of a complete Freund's adjuvant (FCA) or an incomplete Freund's adjuvant (FIA) in the following manner.
First immunization: peptide-KLH 0.5 mg + FCA S.C.
Second to eighth immunizations: peptide-KLH 0.5 mg + FIA S.C.

### (4) Purification of antiserum

Whole blood was drawn from each of the thus-immunized rabbits, and then subjected to centrifugation at 1,600 × g and 4°C for five minutes, to thereby isolate serum.
Antiserum (20 mL) was diluted two-fold with PBS(-) to 40 mL. The diluted antiserum mixture was applied to an antigen peptide column, and an antibody (IgG) was allowed to bind to the antigen, followed by adequate washing with PBS(-) until no absorbance was detected at 280 nm. Thereafter, 0.2 M glycine-HCl (pH 2.5) was caused to pass through the column, to thereby elute the antibody adsorbed onto the antigen. In this case, for pH neutralization, 1 M Tris had been added in advance to a test tube which is employed for receiving the eluted antibody, to thereby prevent denaturation of the thus-collected antibody. The thus-obtained antibody fraction was dialyzed against PBS(-) at 4°C overnight, and the resultant fraction was provided as a purified antibody.

### (5) Determination of specificity of anti-OPRT antibody through western blotting

A homogenate of human lung cancer-derived LC-11 cells (protein concentration: 20 mg/mL) was mixed with an equiamount of a sample preparation liquid for electrophoresis (4% SDS, 10% β-mercaptoethanol, 20% glycerol, 125 mM Tris, pH 6.8), followed by boiling treatment for two minutes. The resultant mixture (10 µL) was subjected to electrophoresis. The sample was electrophoresed on 10% polyacrylamide, and then electrically transferred onto a PVDF filter, followed by blocking through immersion in a blocking agent (Block Ace, product of Dainippon Pharmaceutical Co., Ltd.). Each of the above-produced polyclonal antibodies (serving as a primary antibody), which had been diluted with 20 mM PBS(-) to 1.2 µg/mL, was reacted with the sample for one hour, and the filter was washed with a washing liquid; i.e., 20 mM Tris (pH 7.0) containing 500 mM sodium chloride and 0.5% Tween 20. Thereafter, an alkaline phosphatase-labeled dextran-polymer-bound anti-rabbit polyclonal antibody (Dako Cytomation), serving as a secondary antibody, was reacted with the sample for one hour. Subsequently, the filter was washed with a washing liquid, followed by enzymatic reaction by use of a chemiluminescent reagent (CDP-star, Tropix) for detection of OPRT. The results are shown in Fig. 1. As is clear from Fig. 1, each of the anti-human OPRT antibodies of the present invention specifically recognized only OPRT.

### Example 2 (Quantitative determination of human OPRT through sandwich ELISA)

(1) There was employed, as a standard, rhOPRT prepared through culturing of *Escherichia coli.* The rhOPRT was prepared through the following procedure. The full-length cDNA of human OPRT was cloned through PCR, and a plasmid for expressing a fusion protein of glutathione S-transferase (GST) and rhOPRT was produced. The plasmid was introduced into *Escherichia coli* BL21, and the *Escherichia coli* was subjected to shaking culture overnight in an LB medium (product of Wako Pure Chemical Industries, Ltd.) (100 mL) in the presence of ampicillin (100 µg/mL) at 37°C. The culture (10 mL) was transferred into an Erlenmeyer flask containing an LB medium (1 L), followed by further culturing at 37°C for four hours. Cells were collected through centrifugation, and were suspended in a buffer for collecting cells (50 mM Tris, 8 M urea, 1 mM PMSF, 5 mM EDTA, 5 mM DTT, pH 7.4) (100 mL), followed by gentle stirring at 4°C for 30 minutes. The resultant suspension was subjected to ultrasonic disruption at 4°C, followed by centrifugation at 15,000 x g and 4°C for 30 minutes. Thereafter, the urea concentration of the supernatant was gradually reduced to 1 M through dialysis, followed by refolding treatment. The resultant product was caused to pass through a column charged with glutathione (GSH)-Sepharose (product of Sigma) (2 mL), and washed with a washing liquid (20 mM Tris, 1 M urea, 5 mM EDTA, pH 7.4) (10 mL). Thrombin (600 U) was added to the fusion-protein-bound glutathione (GSH)-Sepharose, and reaction was allowed to proceed in the presence of 2.5 mM calcium chloride at 4°C overnight, to thereby cleave the binding site of the GST-OPRT fusion protein. The resultant thrombin-rhOPRT mixture was caused to pass through a benzamidine-Sepharose column, to thereby yield a target rhOPRT solution (14 mL). The rhOPRT concentration of the solution was found to be 20 µg/mL through protein quantitative determination by the Bradford method.

### (2) Comparison of combinations of anti-OPRT antibodies

Anti-OPRT-A antibody, anti-OPRT-B antibody, or anti-OPRT-C antibody was diluted with 0.1 mM carbonate buffer (pH 9.6) to 5.0 µg/mL, and the thus-diluted antibody (0.1 mL) was dispensed into a 96-well ELISA plate and the plate was sealed. Subsequently, the plate was placed in an incubator at 4°C overnight for coating, whereby an antibody-immobilized support was obtained. The 96-well plate was washed twice with PBS(-) containing 0.05% Tween 20, and subsequently 0.1%-BSA-containing PBS(-) (0.1 mL) was added to the plate for blocking of non-specific adsorption. After the plate had been washed twice with a washing liquid, an rhOPRT solution (concentration: 1.88 ng/mL) (0.1 mL) was added to the plate, to thereby allow reaction to proceed at room temperature for one hour. Subsequently, the plate was washed five times with a washing liquid, and then a peroxidase-labeled anti-OPRT-A, anti-OPRT-B, or anti-OPRT-C antibody which had been diluted with a diluent (0.1% BSA, PBS(-) containing 0.05% Tween 20) to 0.5 µg/mL was dispensed into the plate (0.1 mL/well), to thereby allow reaction to proceed at room temperature for 30 minutes. Subsequently, the plate was washed seven times with a washing liquid, and then 0.1 M phosphate-citrate buffer (pH 5.1) (0.1 mL) containing o-phenylenediamine (1.3 mg/mL), 0.01% aqueous hydrogen peroxide, and 1 mM EDTA, serving as a color-developing liquid, was added to the plate, to thereby allow enzymatic reaction to proceed in the dark at room temperature for 30 minutes. Finally, reaction was stopped by adding 0.1 M sulfuric acid (0.1 mL), and absorbance at 492 nm was measured. The results are shown in Fig. 2.

As is clear from Fig. 2, when anti-OPRT-B antibody was employed, any antibody combination failed to attain sufficient absorbance, whereas when anti-OPRT-A antibody and anti-OPRT-C antibody were employed in combination, rhOPRT level could be determined at the highest sensitivity.

### (3) Quantitative determinability of human OPRT level through sandwich ELISA

Anti-OPRT-C antibody was diluted with 0.1 mM carbonate buffer (pH 9.6) to 5.0 µg/mL, and the thus-diluted antibody (0.1 mL) was dispensed into a 96-well ELISA plate. The plate was sealed and placed in an incubator at 4°C overnight for coating, to thereby yield an antibody-immobilized support. The 96-well plate was washed twice with PBS(-) supplemented with 0.05% Tween 20, and subsequently 0.1%-BSA-containing PBS(-) (0.1 mL) was added to the plate for blocking of non-specific adsorption. After the plate had been washed twice with a washing liquid, an rhOPRT solution (concentration: 0.47, 0.94, 1.88, 3.75, or 7.5 ng/mL) (0.1 mL) was added to the plate, to thereby allow reaction to proceed at room temperature for one hour. Subsequently, the plate was washed five times with a washing liquid, and then a peroxidase-labeled anti-OPRT-A antibody which had been diluted with a diluent to 0.5 µg/mL was dispensed into the plate (0.1 mL/well), to thereby allow reaction to proceed at room temperature for 30 minutes. Subsequently, the plate was washed seven times with a washing liquid, and then a color-developing liquid (i.e., 0.1 M phosphate-citrate buffer (pH 5.1) (0.1 mL) containing o-phenylenediamine (1.3 mg/mL), 0.01% aqueous hydrogen peroxide, and 1 mM EDTA) was added to the plate, to thereby allow enzymatic reaction to proceed in the dark at room temperature for five minutes. Finally, reaction was stopped by adding 0.1 M sulfuric acid (0.1 mL), and absorbance at 492 nm was measured. The results are shown in Fig. 3.

As is clear from Fig. 3, when anti-OPRT-C antibody is employed as an immobilized antibody, and anti-OPRT-A antibody is employed as a detection antibody, rhOPRT level can be determined in a quantitative manner. Thus, a sandwich ELISA system was established.

## Claims

1. An anti-human orotate phosphoribosyltransferase antibody which recognizes an epitope present in a region of 86th to 108th amino acid residues from the N-terminus of human orotate phosphoribosyltransferase.

2. An anti-human orotate phosphoribosyltransferase antibody which recognizes an epitope present in a region of 454th to 474th amino acid residues from the N-terminus of human orotate phosphoribosyltransferase.

3. A human orotate phosphoribosyltransferase protein assay kit comprising an anti-human orotate phosphoribosyltransferase antibody which recognizes an epitope present in a region of 86th to 108th amino acid residues from the N-terminus of human orotate phosphoribosyltransferase; and an anti-human orotate phosphoribosyltransferase antibody which recognizes an epitope present in a region of 454th to 474th amino acid residues from the N-terminus of human orotate phosphoribosyltransferase.

4. The assay kit as described in claim 3, wherein the assay is performed through sandwich ELISA.

5. A method for assaying human orotate phosphoribosyltransferase protein, which method comprises employing, in combination, an anti-human orotate phosphoribosyltransferase antibody which recognizes an epitope present in a region of 86th to 108th amino acid residues from the N-terminus of human orotate phosphoribosyltransferase, and an anti-human orotate phosphoribosyltransferase antibody which recognizes an epitope present in a region of 454th to 474th amino acid residues from the N-terminus of human orotate phosphoribosyltransferase.

6. The assay method as described in claim 5, wherein the assay is performed through sandwich ELISA.
